# EUROPÄISCHE PATENTSCHRIFT

(11) **EP 3 054 782 B1**
(45) Veröffentlichungstag und Bekanntmachung des Hinweises auf die Patenterteilung: **22.05.2019**
(21) Anmeldenummer: 14777620.7
(22) Anmeldetag: 01.10.2014
(51) Int. Cl.: A23K 20/158

(54) **VERFAHREN ZUR TROCKNUNG VON BIOMASSE**
METHOD FOR DRYING BIOMASS
PROCÉDÉ DESTINÉ AU SÉCHAGE DE BIOMASSE

(30) Priorität: 08.10.2013 EP 13187631
(43) Veröffentlichungstag der Anmeldung: 17.08.2016
(73) Patentinhaber: Evonik Degussa GmbH, 45128 Essen (DE)
(72) Erfinder: RABE, Christian, 63762 Großostheim (DE); ALT, Hans Christian, 63571 Gelnhausen (DE); FISCHER, Frank, 65719 Hofheim (DE); EILS, Stefan, 63584 Gründau (DE); PRIEFERT, Horst, 48346 Ostbevern (DE); DIEHL, Michael, 60529 Frankfurt (DE)
(74) Vertreter: Evonik Patent Association
(86) Internationale Anmeldenummer: PCT/EP2014/071002
(87) Internationale Veröffentlichungsnummer: WO 2015/052048

(56) Entgegenhaltungen:
- WO-A2-2008/019887
- DE-A1- 2 651 349
- GB-A- 1 397 410
- US-A- 4 160 040
- US-A1- 2002 110 582
- DATABASE WPI Week 201144 Thomson Scientific, London, GB; AN 2011-F22551 XP002721747, & CN 101 999 522 A (XIAMEN HUISON BIOLOGY CO LTD) 6. April 2011 (2011-04-06)
- DEGUSSA: "Product Information Sipernat D17", INTERNET CITATION, 1. September 2004 (2004-09-01), Seiten 1-2, XP002534705, Gefunden im Internet: URL:http://www.silica-library.com/SilicaWe bAPP/pdf/PI_Sipernatd17_gb.pdf [gefunden am 2009-06-30]
- HIROSHI UEMURA: "Synthesis and production of unsaturated and polyunsaturated fatty acids in yeast: current state and perspectives", APPLIED MICROBIOLOGY AND BIOTECHNOLOGY, SPRINGER, BERLIN, DE, vol. 95, no. 1, 6 May 2012 (2012-05-06), pages 1-12, XP035065847, ISSN: 1432-0614, DOI: 10.1007/S00253-012-4105-1
- CHEN S ET AL: "Whole cell algae powder used for increasing docosahexanoic acid content in milk of high-yielding mammal, comprises docosahexanoic acid-containing algae cell slurry, emulsifier, antioxidant, filler, packaging material, dispersant, and water", WPI / THOMSON,, vol. 2011, no. 44, 6 April 2011 (2011-04-06), XP002721747,
- Bruce G. Hammond ET AL: "Safety Assessment of DHA-Rich Microalgae from Schizochytrium sp.", REGULATORY TOXICOLOGY AND PHARMACOLOGY, vol. 33, no. 2, 1 April 2001 (2001-04-01), pages 192-204, XP55434275, US ISSN: 0273-2300, DOI: 10.1006/rtph.2001.1458

## Beschreibung

Die vorliegende Erfindung betrifft ein Verfahren zur Trocknung einer Biomasse, die einen oxidationsempfindlichen Wertstoff enthält, sowie die durch dieses Verfahren erhältliche Biomasse.

Der Umfang der vorliegenden Erfindung wird nur durch die Ansprüche begrenzt.

Die Bedeutung von mikrobiellen Zellen zur Herstellung von Wertstoffen ist dem Fachmann bekannt. Ein Beispiel für solche Wertstoffe stellen Nahrungsmittelkomponenten dar, insbesondere Lipide wie etwa polyungesättigte Fettsäuren. Für die Herstellung solcher Wertstoffe spielen neben Bakterien und Hefen insbesondere auch weitere Pilze sowie Algen eine besondere Rolle.

Bestimmte Wertstoffe, insbesondere polyungesättigte Fettsäuren (PUFAs), stellen eine wichtige Komponente für die Ernährung von Mensch und Tier dar. Als Quelle für omega-3-Fettsäuren wurde anfangs vor allem Fisch verwendet. Später wurde entdeckt, dass bestimmte Mikroben heterotroph omega-3-Fettsäuren in großen Mengen produzieren, wobei die Fettsäureproduktion durch Wahl spezifischer Reaktionsparameter vorteilhaft beeinflusst werden kann. Die omega-3-Fettsäuren können anschließend aus den Zellen gewonnen werden oder aber die Zellen in Form von Biomasse direkt in Futter- oder Nahrungsmitteln eingesetzt werden.

Um die Biomasse für den Einsatz in Futter- oder Nahrungsmitteln oder für die nachfolgende Isolation des Wertstoffs aufzubereiten, ist es erforderlich, diese in eine gut handhabbare, fließfähige Form zu überführen.

Es hat sich jedoch herausgestellt, dass die im Stand der Technik beschriebene Aufarbeitung der Biomasse häufig zu einem schlecht handhabbaren, nicht fließfähigen, in der Regel hygroskopischen Produkt führt. Dies ist vor allem dann der Fall, wenn die Biomasse einen hohen Anteil an Lipiden, insbesondere Triglyceriden, enthält.

Aufgabe der vorliegenden Erfindung war es daher, ein Verfahren zur Verfügung zu stellen, das die Überführung der erhaltenen Biomasse in ein besser handhabbares, fließfähiges, nicht hygroskopisches definiertes partikuläres Produkt erlaubt.

Bei der Aufarbeitung sollte insbesondere auch der erhaltene Wertstoff weitestgehend in intakter Form erhalten bleiben. Insofern sollte die Aufarbeitung insbesondere auch sicherstellen, dass die Zellmembranen der in der Biomasse enthaltenen Zellen weitestgehend in intakter Form erhalten bleiben, um somit den oxidativen Abbau des enthaltenen Wertstoffs zu verhindern. Weiterhin sollte vorzugsweise auch so weit wie möglich verhindert werden, dass gegebenenfalls freigesetzter Wertstoff, beispielsweise durch oxidativen Abbau, geschädigt wird.

Erfindungsgemäß hat sich herausgestellt, dass die erfindungsgemäße Aufgabe dadurch gelöst werden kann, dass bei der Aufarbeitung der die Biomasse enthaltenen Fermentationsbrühe zu einer partikulären Zusammensetzung eine Kieselsäure als Additiv verwendet wird, wobei sich die Verwendung hydrophiler und hydrophober Kieselsäuren als besonders vorteilhaft herausgestellt hat.

Die Verwendung der Kieselsäure bei der Herstellung der partikulären Zusammensetzung ausgehend von der Fermentationsbrühe führt zu einem klar definierten, sehr gut handhabbaren, fließfähigen, nicht-hygroskopischen Produkt, das sich sowohl sehr gut zur Einarbeitung in Futter- oder Nahrungsmitteln eignet, sehr gut zu Partikeln mit definierten Teilchendurchmesser weiterverarbeitet werden kann sowie ebenfalls sehr gut für die Isolation des enthaltenen Wertstoffs aus diesem Produkt verwendet werden kann.

Weiterhin wird gegebenenfalls freigesetzter Wertstoff durch die Kieselsäure absorbiert und dadurch vor oxidativem Abbau geschützt.

WO 2008/019887 offenbart ein Verfahren zur Herstellung einer partikulären Biomasse, die eine Mischung aus Hefe oder Lactobacillen mit hydrophober Kieselsäure (Sipernat D17) umfasst. Die Mischung wird hergestellt durch Sprühtrocknung und kann als Tierfutter eingesetzt werden.

CN 101999522 offenbart ein Verfahren zur Herstellung von partikulärer omega-3 Fettsäuren enthaltender Biomasse, die eine Mischung aus Algenpulver und Kieselsäure umfasst. Die Mischung wird hergestellt durch Sprühtrocknung und wird als Tierfutter eingesetzt. Der Einsatz von hydrophober oder hydrophiler Kieselsäure wird in CN 101999522 nicht beschrieben.

US 2002/110582 offenbart ein Verfahren zur Herstellung von partikulärer omega-3 Fettsäuren enthaltender Biomasse, die eine Mischung aus Algenpulver, insbesondere Schizochytrien-Zellen, und Kieselsäure umfasst. Aus Hammond et al., Regulatory Toxicology and Pharmacology, 2001, Bd. 33(2), 192-204, geht hervor, dass Schizochytrium-Zellen 41 % Fett aufweisen können. Das Produkt wird als Futter in der Aquakultur eingesetzt. Der Einsatz von hydrophober oder hydrophiler Kieselsäure wird in US 2002/110582 nicht beschrieben.

Ein erster Gegenstand der vorliegenden Erfindung ist daher die Herstellung einer partikulären rieselfähigen Biomasse, die einen oxidationsempfindlichen Wertstoff enthält, dadurch gekennzeichnet, dass bei der Herstellung der partikulären Biomasse eine Kieselsäure als Additiv eingesetzt wird, wobei es sich bei der Kieselsäure um eine hydrophile oder eine hydrophobe Kieselsäure handelt und wobei die Kieselsäure in einer Menge eingesetzt wird, so dass sich in der partikulären Biomasse eine Konzentration an Kieselsäure von 0,1 bis 8 Gew.-% einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen.

Ein bevorzugter Gegenstand der vorliegenden Erfindung ist daher die Herstellung einer partikulären rieselfähigen Biomasse, die einen oxidationsempfindlichen Wertstoff enthält, dadurch gekennzeichnet, dass bei der Herstellung der partikulären Biomasse eine hydrophile Kieselsäure als Additiv eingesetzt wird, wobei die hydrophile Kieselsäure in einer Menge eingesetzt wird, so dass sich in der partikulären Biomasse eine Konzentration an hydrophiler Kieselsäure von 0,1 bis 8 Gew.-% einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen.

Weiterer bevorzugter Gegenstand der vorliegenden Erfindung ist daher auch die Herstellung einer partikulären rieselfähigen Biomasse, die einen oxidationsempfindlichen Wertstoff enthält, dadurch gekennzeichnet, dass bei der Herstellung der partikulären Biomasse eine hydrophobe Kieselsäure als Additiv eingesetzt wird, wobei die hydrophobe Kieselsäure in einer Menge eingesetzt wird, so dass sich in der partikulären Biomasse eine Konzentration an hydrophober Kieselsäure von 0,1 bis 8 Gew.-% einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen.

Weiterer Gegenstand der vorliegenden Erfindung ist daher auch eine partikuläre rieselfähige Biomasse, die einen oxidationsempfindlichen Wertstoff enthält, dadurch gekennzeichnet, dass die partikuläre Biomasse, vorzugsweise eine hydrophile Kieselsäure oder eine hydrophobe Kieselsäure in einer Menge von 0,1 bis 8 Gew.-%, vorzugsweise 0,2 bis 6 Gew.-%, besonders bevorzugt 0,5 bis 2,5 Gew.-%, vor allem 0,5 bis 2,5 Gew-%, insbesondere 0,5 bis 1,5 Gew.-% oder 0,5 bis 1 Gew.-%, enthält, wobei die Zellen der Biomasse einen Lipid-Gehalt von mindestens 20 Gew.-%, bezogen auf die Zelltrockenmasse, aufweisen und wobei die Biomasse Zellen des Taxons Labyrinthulomycetes umfasst.

Ein bevorzugter Gegenstand der vorliegenden Erfindung ist daher auch eine partikuläre rieselfähige Biomasse, die einen oxidationsempfindlichen Wertstoff enthält, dadurch gekennzeichnet, dass die partikuläre Biomasse eine hydrophile Kieselsäure in einer Menge von 0,1 bis 8 Gew.-%, vorzugsweise 0,2 bis 6 Gew.-%, besonders bevorzugt 0,5 bis 2,5 Gew.-%, vor allem 0,5 bis 2,5 Gew-%, insbesondere 0,5 bis 1,5 Gew.-% oder 0,5 bis 1 Gew.-%, enthält, wobei die Zellen der Biomasse einen Lipid-Gehalt von mindestens 20 Gew.-%, bezogen auf die Zelltrockenmasse, aufweisen und wobei die Biomasse Zellen des Taxons Labyrinthulomycetes umfasst.

Ein bevorzugter Gegenstand der vorliegenden Erfindung ist daher ebenso eine partikuläre rieselfähige Biomasse, die einen oxidationsempfindlichen Wertstoff enthält, dadurch gekennzeichnet, dass die partikuläre Biomasse eine hydrophobe Kieselsäure in einer Menge von 0,1 bis 8 Gew.-%, vorzugsweise 0,2 bis 6 Gew.-%, besonders bevorzugt 0,5 bis 2,5 Gew.-%, vor allem 0,5 bis 2,5 Gew-%, insbesondere 0,5 bis 1,5 Gew.-% oder 0,5 bis 1 Gew.-%, enthält, wobei die Zellen der Biomasse einen Lipid-Gehalt von mindestens 20 Gew.-%, bezogen auf die Zelltrockenmasse, aufweisen und wobei die Biomasse Zellen des Taxons Labyrinthulomycetes umfasst.

Weiterer Gegenstand der vorliegenden Erfindung ist daher auch ein Nahrungs- oder Futtermittel, dadurch gekennzeichnet, dass es eine erfindungsgemäße partikuläre Biomasse umfasst.

Die Herstellung der partikulären rieselfähigen Biomasse erfolgt erfindungsgemäß vorzugsweise ausgehend von einer Fermentationsbrühe, die die Biomasse enthält.

Zur Überführung der Biomasse aus der Fermentationsbrühe in eine partikuläre rieselfähige Zusammensetzung muss eine Trocknung der Biomasse erfolgen. Vor der eigentlichen Trocknung der Biomasse kann auch zunächst eine Einengung der die Biomasse enthaltenden Fermentationsbrühe erfolgen, um den Feststoffgehalt vor der eigentlichen Trocknung zu erhöhen. Eine vorherige Einengung ist jedoch nicht zwingend erforderlich.

Die Trocknung der Fermentationsbrühe zu einer partikulären Zusammensetzung erfolgt erfindungsgemäß vorzugsweise durch ein thermisches Verfahren, insbesondere durch Sprühtrocknung, Trommeltrocknung oder Wirbelschichtgranulation.

Die Kieselsäure kann gegebenenfalls bereits in der Fermentationsbrühe enthalten sein oder aber vor der Einengung und/oder vor der Trocknung der Fermentationsbrühe zugegeben werden. Alternativ kann die Kieselsäure auch erst nach der Trocknung in einem weiteren Verfahrensschritt zu der Biomasse hinzugegeben werden.

In einer bevorzugten Ausführungsform wird die Kieselsäure während des Trocknungsschrittes bzw. im Verlauf des Trocknungsverfahrens mit der Fermentationsbrühe bzw. der noch feuchten Biomasse vermischt. Die Kieselsäure wird hierbei vorzugsweise unter Verwendung einer Düse, vorzugsweise einer Zweistoffdüse, in die Trocknungszone dosiert. Die Kieselsäure kann hierbei in suspendierter Form dosiert werden, wird jedoch bevorzugt in trockener, insbesondere pulverförmiger, Form dosiert.

Die Trocknungszone, in welche die Kieselsäure vorzugsweise dosiert wird, stellt bei der Wirbelschichtgranulation die Wirbelschicht dar. Bei der Düsensprühtrocknung ist die Trocknungszone entsprechend die Zone unterhalb der Einsprühdüse, durch welche die Fermentationsbrühe zudosiert wird. Bei der Fließbettgranulation und Trommeltrocknung ist die Trocknungszone entsprechend das Fließbett bzw. die Trommel.

Alternativ kann die Kieselsäure auch teilweise bereits in der Fermentationsbrühe enthalten sein und zusätzlich weitere Kieselsäure im Verlauf des Trocknungsverfahrens zudosiert werden.

In einer weiteren bevorzugten Ausführungsform wird die Kieselsäure erst nach dem Trocknungsschritt, also erst nach der Trocknung der Biomasse durch vorzugsweise Sprühtrocknung, Trommeltrocknung oder Wirbelschichtgranulation, in einem zusätzlichen Verfahrensschritt mit der Kieselsäure umgesetzt. Dies erfolgt vorzugsweise in einem mechanischen Mischvorgang, beispielsweise unter Verwendung eines kontinuierlichen Mischers oder eines Batch-Mischers.

Hydrophile Kieselsäuren sind unter der CAS-Nr. 112926-00-8 registriert und kommerziell beispielsweise unter dem Handelsnamen Sipernat® (Evonik Industries, Deutschland) erhältlich.

Erfindungsgemäß bevorzugt eingesetzte hydrophile Kieselsäuren haben eine spezifische Oberfläche (ISO 9277) von 130 bis 600 m²/g, vorzugsweise 160 bis 550 m²/g, und vorzugsweise einen Dioctyladipat-Absorptionswert von 1,5 - 4,0 ml/g, vorzugsweise 2,0 - 3,2 ml/g.

Sie besitzen ferner vorzugsweise eine Stampfdichte (nicht gesiebt; in Anlehnung and ISO 787-11) von 80 bis 300 g/l, vorzugsweise 100 bis 270 g/l.

Die Partikelgröße der hydrophilen Kieselsäure (d50; Laserbeugung; in Anlehnung an ISO 13320-1) beträgt vorzugsweise 10 bis 150 µm, insbesondere 15 bis 130 µm.

Der Trocknungsverlust der hydrophilen Kieselsäure (2 Stunden bei 105°C; in Anlehnung an ISO 787-2) beträgt vorzugsweise maximal 10 %, besonders bevorzugt maximal 7 %.

Der Glühverlust der hydrophoben Kieselsäure (2 Stunden bei 1000°C; in Anlehnung an ISO 3262-1) beträgt vorzugsweise maximal 10 %, besonders bevorzugt maximal 6 %.

Der Siliciumdioxid-Gehalt der hydrophilen Kieselsäure beträgt vorzugsweise mindestens 95 Gew.-%, besonders bevorzugt mindestens 97 Gew.-% (in Anlehnung an ISO 3262-19).

Der pH-Wert der hydrophilen Kieselsäure (5 % in Wasser; in Anlehnung an ISO 787-9) beträgt vorzugsweise von 5,0 bis 7,0, besonders bevorzugt von 6,0 bis 6,5.

In einer erfindungsgemäß besonders bevorzugten Ausführungsform handelt es sich bei der hydrophilen Kieselsäure um ein Produkt mit einer spezifischen Oberfläche von 160 bis 220 m²/g, einem Dioctyladipat-Absorptionswert von 2,0 - 2,8 ml/g, einer Stampfdichte von 200 bis 300 g/l und einer Partikelgröße von 100 bis 150 µm. Ein solches Produkt ist unter dem Namen Sipernat® 22 S (Evonik Industries, Deutschland) im Handel erhätlich.

In einer weiteren erfindungsgemäß besonders bevorzugten Ausführungsform handelt es sich bei der hydrophilen Kieselsäure um ein Produkt mit einer spezifischen Oberfläche von 450 bis 550 m²/g, einem Dioctyladipat-Absorptionswert von 2,5 - 3,5 ml/g, einer Stampfdichte von 80 bis 130 g/l und einer Partikelgröße von 10 bis 40 µm. Ein solches Produkt ist unter dem Namen Sipernat® 50 S (Evonik Industries, Deutschland) im Handel erhältlich.

Hydrophobe Kieselsäuren sind unter der CAS-Nr. 68611-44-9 registriert und kommerziell beispielsweise ebenfalls unter dem Handelsnamen Sipernat® (Evonik Industries, Deutschland) erhältlich.

Erfindungsgemäß bevorzugt eingesetzte hydrophobe Kieselsäuren besitzen eine Methanolbenetzbarkeit von mindestens 40 %, vorzugsweise mindestens 45 %, besonders bevorzugt mindestens 50 %, insbesondere 40 bis 65 %, vor allem 50 bis 60 %.

Sie besitzen ferner vorzugsweise eine Stampfdichte (nicht gesiebt; in Anlehnung an ISO 787-11) von 100 bis 200 g/l, vorzugsweise 125 bis 175 g/l.

Die Partikelgröße der hydrophoben Kieselsäure (d50; Laserbeugung; in Anlehnung an ISO 13320-1) beträgt vorzugsweise 5 bis 15 µm, insbesondere 8 bis 12 µm.

Der Trocknungsverlust der hydrophoben Kieselsäure (2 Stunden bei 105°C; in Anlehnung an ISO 787-2) beträgt vorzugsweise maximal 10 %, besonders bevorzugt maximal 6 %.

Der Glühverlust der hydrophoben Kieselsäure (2 Stunden bei 1000°C; in Anlehnung an ISO 3262-1) beträgt vorzugsweise maximal 10 %, besonders bevorzugt maximal 6 %.

Der Siliciumdioxid-Gehalt der hydrophoben Kieselsäure beträgt vorzugsweise mindestens 95 Gew.-%, besonders bevorzugt mindestens 97 Gew.-% (in Anlehnung an ISO 3262-19).

Der Kohlenstoffgehalt der hydrophoben Kieselsäure beträgt vorzugsweise maximal 3,5 Gew.-%, insbesondere maximal 2 Gew.-% (in Anlehnung an ISO 3262-19).

Der pH-Wert der hydrophoben Kieselsäure (5 % in einem 1:1-Gemisch aus Wasser und Methanol; in Anlehnung an ISO 787-9) beträgt vorzugsweise von 7 bis 10,5, besonders bevorzugt von 7,5 bis 9.

Die Methanolbenetzbarkeit stellt ein Maß für die Hydrophobie des Kieselsäurepulvers dar. Zur Bestimmung dieses Wertes wird eine bestimmte Menge Kieselsäurepulver in Wasser eingewogen. Dabei bleibt das Kieselsäurepulver an der Oberfläche. Es wird nun die zur Benetzung des Pulvers notwendige Methanol-Menge bestimmt. Unter "Methanolbenetzbarkeit" wird hierbei der Methanolgehalt einer Methanol-Wasser-Mischung in Volumen-% verstanden, bei welchem 50 % der hydrophoben Kieselsäure sedimentieren.

Erfindungsgemäß einsetzbare hydrophobe Kieselsäuren sind beispielsweise unter den Handelsnamen Sipernat® D 10, Sipernat® D 15 und Sipernat® D 17 (Evonik Industries, Deutschland) erhältlich.

Die Kieselsäure wird der Biomasse bzw. Fermentationsbrühe in einer Menge zudosiert, so dass sich im finalen getrockneten Produkt eine Konzentration von 0,1 bis 8,0 Gew.-%, insbesondere 0,1 bis 6,0 Gew.-%, besonders bevorzugt 0,2 bis 2,5 Gew.-%, vor allem 0,5 bis 2,5 Gew.-%, insbesondere 0,5 bis 1,5 Gew.-% oder 0,5 bis 1 Gew.-%, einstellt.

Die Fermentationsbrühe besitzt vor Beginn der eigentlichen Trocknung vorzugsweise einen Feststoffgehalt von 10 bis 50 Gew.-% und entsprechend einen Wassergehalt von 50 bis 90 Gew.-%. Sofern erforderlich wird die Fermentationsbrühe vor der eigentlichen Trocknung auf diesen Wassergehalt eigestellt.

Unter "Feststoffgehalt" ist erfindungsgemäß die Masse zu verstehen, die bei vollständigem Entzug des Wassers zurückbleibt. Zu dieser Trockenmasse gehören neben gegebenenfalls suspendierten Substanzen (wie der Biomasse) auch gelöste Substanzen, die erst bei der Trocknung auskristallisieren oder ausfällen. Der Feststoffgehalt ist insofern komplementär zum Wasser- bzw. Feuchtigkeitsgehalt.

Die bei der Trocknung eingesetzte, Biomasse enthaltende Zusammensetzung ist vorzugsweise das Produkt eines fermentativen Kultivierungsverfahrens und wird entsprechend auch als Fermentationsbrühe bezeichnet. Die erfindungsgemäß einzusetzende Fermentationsbrühe umfasst neben der zu trocknenden Biomasse vorzugsweise weitere Bestandteile des Fermentationsmediums. Bei diesen Bestandteilen kann es sich insbesondere um Salze, Antischaummittel und nicht umgesetzte Kohlenstoffquelle und/oder Stickstoffquelle handeln. Bei der Trocknung entsteht ein Produkt, das einen Zellgehalt von mindestens 60 Gew.-%, vorzugsweise mindestens 65 Gew.-%, insbesondere mindestens 70 oder 80 Gew.-%, aufweist, wobei als
weitere Bestandteile die Kieselsäure und gegebenenfalls die zuvor genannten weiteren Bestandteile des Fermentationsmediums sowie gegebenenfalls teilweise aus den Zellen freigesetzte Komponenten enthalten sind. Die weiteren Bestandteile der Fermentationsbrühe können gegebenenfalls vor der Trocknung der Biomasse teilweise abgetrennt werden, beispielsweise durch Fest-Flüssig-Trennverfahren, so dass bei der Trocknung ein Produkt entsteht, dass diese weiteren Komponenten der Fermentationsbrühe, insbesondere Salze, vorzugsweise in einer Menge von maximal 20 Gew.-%, insbesondere maximal 15, 10 oder 5 Gew.-%, enthält.

Bei den in der Biomasse enthaltenen Zellen handelt es sich um Zellen, die einen oxidationsempfindlichen Wertstoff enthalten. Es kann sich hierbei insbesondere um Zellen handeln, die bereits natürlicherweise Wertstoffe, vorzugsweise Lipide, insbesondere PUFAs (polyungesättigte Fettsäuren), produzieren, es kann sich jedoch auch um Zellen handeln, die durch entsprechende gentechnische Verfahren dazu in die Lage versetzt wurden, Lipide, insbesondere PUFAs, zu produzieren. Die Produktion kann hierbei autotroph, mixotroph oder heterotroph erfolgen.

Bevorzugt umfasst die Biomasse Zellen, die Lipide, insbesondere PUFAs, heterotroph produzieren. Es handelt sich bei den Zellen erfindungsgemäß vorzugsweise um Algen, Pilze, insbesondere Hefen, oder Protisten. Besonders bevorzugt handelt es sich um Zellen mikrobieller Algen oder Pilze.

Als Zellen von öl-produzierenden Hefen kommen insbesondere Stämme von Yarrowia, Candida, Rhodotorula, Rhodosporidium, Cryptococcus, Trichosporon und Lipomyces in Betracht.

Erfindungsgemäß umfasst die Biomasse vorzugsweise Zellen des Taxons Labyrinthulomycetes (Labyrinthulea, Netzschleimpilze, Schleimnetze), insbesondere solche der Familie der Thraustochytriaceae. Zu der Familie der Thraustochytriaceae gehören die Gattungen Althomia, Aplanochytrium, Elnia, Japonochytrium, Schizochytrium, Thraustochytrium, Aurantiochytrium, Oblongichytrium und Ulkenia. Besonders bevorzugt umfasst die Biomasse Zellen der Gattungen Thraustochytrium, Schizochytrium, Aurantiochytrium oder Oblongichytrium, vor allem solche der Gattung Aurantiochytrium.

Innerhalb der Gattung Aurantiochytrium ist erfindungsgemäß die Spezies Aurantiochytrium limacinum (früher auch Schizochytrium limacinum genannt) bevorzugt. Ganz besonders bevorzugt wird erfindungsgemäß der Stamm Aurantiochytrium limacinum SR21 ((IFO 32693) eingesetzt.

Bei dem oxidationsempfindlichen Wertstoff handelt es sich vorzugsweise um ein oxidationsempfindliches Lipid, insbesondere um eine ungesättigte Fettsäure, besonders bevorzugt um eine polyungesättigte Fettsäure (PUFA) oder hochungesättigte Fettsäure (HUFA).

Die in der Biomasse enthaltenen Zellen zeichnen sich dadurch aus, dass sie einen Lipid-Gehalt, besonders bevorzugt PUFA-Gehalt, von mindestens 20 Gew.-%, vorzugsweise mindestens 30 Gew.-%, insbesondere mindestens 40 Gew.-%, jeweils bezogen auf die Zelltrockenmasse, aufweisen.

In einer bevorzugten Ausführungsform liegt hierbei ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Weiterhin umfassen die in der Zelle enthaltenen Lipide vorzugsweise polyungesättigte Fettsäuren (PUFAs), wobei vorzugsweise mindestens 10 Gew.-%, insbesondere mindestens 20 Gew.-%, besonders bevorzugt 20 bis 60 Gew.-%, insbesondere 20 bis 40 Gew.-%, der in der Zelle enthaltenen Fettsäuren PUFAs darstellen.

Unter polyungesättigten Fettsäuren (PUFAs) werden erfindungsgemäß Fettsäuren verstanden, die mindestens zwei, insbesondere mindestens drei, C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5Z,8Z,11Z,14Z,17Z)-Eicosa-5,8,11,14,17-pentaensäure, und die Docosahexaensäure (DHA, 22:6ω-3), insbesondere die (4Z,7Z,10Z,13Z,16Z,19Z)-Docosa-4,7,10,13,16,19-hexaensäure, dar, wobei die Docosahexaensäure besonders bevorzugt ist.

Verfahren zur Herstellung der Biomasse, insbesondere solcher Biomasse, die Lipide, insbesondere PUFAs, enthaltende Zellen, insbesondere der Ordnung Thraustochytriales, umfasst, sind im Stand der Technik ausführlich beschrieben. Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden. Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon.

Die Fermentation der Zellen erfolgt vorzugsweise in einem Medium mit niedriger Salinität, insbesondere um Korrosion zu verhindern. Dies kann dadurch erreicht werden, dass anstelle von Natriumchlorid chlor-freie Natriumsalze, wie beispielsweise Natriumsulfat, Natriumcarbonat, Natriumhydrogencarbonat oder Sodaasche, als Natriumquelle verwendet werden. Vorzugsweise wird Chlorid in Mengen von weniger als 3 g/l, insbesondere weniger als 500 mg/l, besonders bevorzugt weniger als 100 mg/l, bei der Fermentation eingesetzt.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Zusätzlich können auch anorganische oder organische Phosphorverbindungen und/oder bekannte wachstumsstimulierende Stoffe, wie etwa Hefeextrakt oder Maisquellwasser, hinzugegeben werden, um die Fermentation positiv zu beeinflussen.

Die Fermentation der Zellen erfolgt vorzugsweise bei einem pH-Wert von 5 bis 11, insbesondere 6 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Nach Beendigung der Fermentation erfolgt die Ernte der Biomasse. Vorzugsweise erfolgt nach der Ernte der Biomasse oder gegebenenfalls auch schon kurz vor der Ernte der Biomasse eine Pasteurisierung der Zellen, um die Zellen abzutöten und Enzyme, die den Abbau der Lipide befördern könnten, zu inaktivieren. Die Pasteurisierung erfolgt vorzugsweise durch Erhitzen der Biomasse auf eine Temperatur von 50 bis 121 °C für eine Dauer von 5 bis 60 Minuten.

Ebenso erfolgt nach der Ernte der Biomasse oder gegebenenfalls auch schon kurz vor der Ernte der Biomasse vorzugsweise die Zugabe von Antioxidantien, um den in der Biomasse enthaltenen Wertstoff vor oxidativem Abbau zu schützen. Bevorzugte Antioxidantien stellen hierbei BHT, BHA, TBHA, Ethoxyquin, beta-Carotin, Vitamin E und Vitamin C dar. Das Antioxidans wird, sofern eingesetzt, vorzugsweise in einer Menge von 0,01 bis 2 Gew.-% hinzugegeben.

Gegebenenfalls kann nach Beendigung der Fermentation auch bereits Kieselsäure in die Fermentationsbrühe zugegeben werden. Es handelt sich hierbei jedoch um eine weniger bevorzugte Ausführungsform. Denn es hat sich als vorteilhaft herausgestellt, die Kieselsäure erst während der eigentlichen Trocknung oder sogar erst nach der eigentlichen Trocknung hinzuzugegeben.

Gegebenenfalls kann nun, vor der eigentlichen Trocknung, bereits ein Teil des Fermentationsmediums von der Biomasse abgetrennt und damit der Feststoffanteil erhöht werden. Dies kann insbesondere durch Zentrifugation, Flotation, Filtration, insbesondere Ultra- oder Mikrofiltration, Dekantieren und/oder Lösungsmittelverdampfung erfolgen. Die Lösungsmittelverdampfung erfolgt hierbei vorzugsweise unter Einsatz eines Rotationsverdampfers, eines Dünnschichtverdampfers oder eines Fallfilmverdampfers in einem einstufigen oder mehrstufigen Prozess. Alternativ kommt beispielsweise auch die Umkehrosmose zwecks Einengung der Fermentationsbrühe in Betracht.

In diesem ersten optionalen, aber bevorzugt durchgeführten, Schritt erfolgt vorzugsweise eine Aufkonzentrierung der Fermentationsbrühe auf einen Feststoffgehalt von mindestens 10 oder 15 Gew.-%, vorzugsweise von mindestens 20 oder 25 Gew.-%, insbesondere 10 bis 50 oder 15 bis 45 Gew.-%, besonders bevorzugt 15 bis 40 Gew.-% oder 20 bis 40 Gew.-%.

D.h. die in einem erfindungsgemäßen Verfahren zu trocknende Biomasse liegt vorzugsweise in Form einer Suspension mit dem zuvor angegebenen Feststoffanteil vor, wobei es sich bei der Suspension vorzugsweise um Fermentationsbrühe oder eingeengte Fermentationsbrühe handelt.

Nach der optionalen Einengung der Fermentationsbrühe erfolgt nun die erfindungsgemäße Trocknung der Biomasse, vorzugsweise durch Sprühtrocknung, insbesondere Düsensprühtrocknung, Sprühgranulation, Wirbelschichtgranulation, insbesondere Fließbettgranulation, oder in einem Trommeltrockner.

Alternativ kann die Biomasse auch direkt nach der Ernte ohne vorherige Einengung dem Trocknungsschritt unterzogen werden, insbesondere dann, wenn die erhaltene Fermentationsbrühe bereits einen hohen Feststoffgehalt, vorzugsweise wie zuvor angegeben, aufweist.

Durch die Trocknung der Biomasse wird diese vorzugsweise auf eine Restfeuchte von maximal 10 Gew.-%, insbesondere 0 bis 10 Gew.-%, besonders bevorzugt maximal 8 Gew.-%, insbesondere 0,5 bis 8 Gew.-%, vor allem maximal 6 oder 5 Gew.-%, insbesondere 0,5 bis 6 oder 0,5 bis 5 Gew.-%, getrocknet.

Vorzugsweise befinden sich nach der Trocknung mindestens 50 %, insbesondere mindestens 60 oder 70 %, vor allem mindestens 80 oder 90 %, der in der Biomasse enthaltenen Zellen im intakten Zustand. Die Anzahl der intakten Zellen kann beispielsweise visuell unter Verwendung eines Mikroskops bestimmt werden als das Verhältnis der Anzahl intakter Zellen in Bezug zur Gesamtzahl der Zellen.

Die erfindungsgemäße Trocknung der Biomasse erfolgt in einer erfindungsgemäß besonders bevorzugten Ausführungsform in einem Wirbelschichtgranulationsverfahren oder einem Düsensprühtrocknungsverfahren. Die Biomasse enthaltende Fermentationsbrühe sowie gegebenenfalls die Kieselsäure werden hierzu vorzugsweise in die jeweilige Trocknungszone eingesprüht.

In die Wirbelschicht-Trocknungsgranulationsanlage wird das Trocknungsgas von unten eingeleitet. Der Großteil der Feuchtigkeit der eingesprühten Fermentationsbrühe verdampft und das entstehende Granulat wird durch den Gasstrom des Trocknungsgases in der Schwebe gehalten. In diesem Zustand sind die Partikel voneinander getrennt und so beim Einsprühen von weiterer Flüssigkeit in das Bett für die Sprühtropfen frei zugänglich. Außerdem ist in diesem Zustand der Wärme- und Stoffaustausch zwischen den Feststoffpartikeln und dem Gasstrom intensiv. Produktpartikel mit der gewünschten Größe werden kontinuierlich in einem klassierenden Abzug aus der Wirbelschicht abgezogen.

Die Wirbelschicht oder das Bett aus Partikeln, welches zu Beginn der WirbelschichtGranulationstrocknung vorhanden sein muss, besteht vorzugsweise aus einer Mischung von Partikeln der Kieselsäure sowie getrockneter Partikeln der zur Trocknung eingesetzten Biomasse, beispielsweise aus einer Charge eines vorherigen Laufs. Es ist jedoch genauso gut möglich, einen anderen Stoff als Wirbelschicht zur Initiierung der WirbelschichtGranulationstrocknung einzusetzen.

Zusätzlich werden während der Wirbelschichtgranulation vorzugsweise feine Partikel (<100 µm) der getrockneten Biomasse zudosiert. Dies geschieht vorzugsweise im Verhältnis eins zu eins relativ zum Feststoff, der mit der Fermentationsbrühe in die Trocknungszone gelangt. Diese feinen Partikel können aus einer gleichzeitig stattfindenden Vermahlung des fertigen Produktes generiert werden. Alternativ können diese feinen Partikel auch in einem vorgelagerten Verfahrensschritt der Sprühtrocknung erzeugt werden.

In der Düsensprühtrocknung wird die eingeleitete Fermentationsbrühe in eine definierte Tropfengröße zerstäubt und mit der eingeleiteten Trocknungsluft im Gleichstrom getrocknet. Da auch in diesem Verfahren die einzelnen Tropfen voneinander getrennt sind, besteht ein guter Wärme- und Stofftransport und somit eine effiziente Trocknung. Zudem kann über die eingestellte Tropfengröße in der Düse die Partikelgröße des getrockneten Endprodukts definiert eingestellt werden und somit ist kein Klassiervorgang notwendig.

In beiden vorteilhaften Trocknungsverfahren wird die Kieselsäure in der Trocknungszone zerstäubt, um ein Aneinanderheften der Trocknungspartikel zu vermeiden. Die Kieselsäure fungiert somit als sogenanntes Antibackmittel, wodurch das Einstellen einer definierten und regelbaren Partikelgröße möglich wird.

Das Trocknungsgas wird in beiden Trocknungsverfahren vorzugsweise in Kreisgasfahrweise über die Biomasse geleitet. "Kreisgasfahrweise" bedeutet, dass das zur Trocknung verwendete Gas zirkulierend über die Biomasse geleitet wird. Das bei der Trocknung eingesetzte Gas hat vorzugsweise eine Temperatur oberhalb der Sättigungstemperatur des zu verdampfenden Lösungsmittels. Bei dem eingesetzten Gas handelt es sich vorzugsweise um Luft, vorzugsweise um Luft mit reduziertem Gehalt an Sauerstoff.

Das in Kreisgasfahrweise geführte Gas weist vorzugsweise einen Sauerstoffgehalt von weniger als 20 Gew.-%, vorzugsweise weniger als 15 Gew.-%, insbesondere von 5 bis 13 Gew.-%, auf.

Das Gas wir vorzugsweise dadurch erzeugt, das Luft über einen Brenner geleitet und auf diese Weise erhitzt wird. Zugleich wird hierdurch der Sauerstoffgehalt der Luft auf unter 20 Gew.-%, vorzugsweise auf weniger als 15 Gew.-%, insbesondere auf 5 bis 13 Gew.-% reduziert. Das Gas wird stets auf gleiche Weise nachreguliert, um einen konstanten Gasstrom mit vermindertem Sauerstoffgehalt zu erzeugen.

In beiden Verfahren wird die in der Fermentationsbrühe enthaltene Biomasse unmittelbar in ein Granulat überführt. Ein besonderer Vorteil dieser Verfahren besteht somit darin, dass die in der Fermentationsbrühe enthaltene Biomasse in einem einzigen Schritt zu einem Produkt gewünschter Korngröße getrocknet werden kann und somit nur ein Verfahrensschritt von der Biomasse enthaltenden Fermentationsbrühe bis zum fertigen Produkt erforderlich ist.

Ein weiterer Vorteil dieser Verfahren besteht darin, dass sie kontinuierlich und stationär betrieben werden können: Biomasse enthaltende Fermentationsbrühe sowie Kieselsäure können kontinuierlich eingesprüht werden und das fertige Produkt kann kontinuierlich ausgetragen werden.

Die Wirbelschicht bei der Wirbelschichtgranulation hat vorzugsweise eine Temperatur von 45 bis 95 °C, insbesondere 45 bis 75 °C, besonders bevorzugt 50 bis 60 °C. Die Luft wird entsprechend so stark erhitzt, dass sich in der Wirbelschicht eine Temperatur von 45 bis 95 °C, insbesondere 45 bis 75 °C, besonders bevorzugt 50 bis 60 °C, einstellt.

Die Trocknungstemperatur im Düsensprühturm kann aufgrund der kurzen Verweilzeiten auf 95°C eingestellt werden
Besonders bevorzugt ist erfindungsgemäß ein Verfahren zur Herstellung einer partikulären Biomasse, die einen oxidationsempfindlichen Wertstoff enthält, ausgehend von einer Fermentationsbrühe, dadurch gekennzeichnet, dass die Fermentationsbrühe einer Düsensprühtrocknung oder einer Wirbelschichtgranulationstrocknung unterzogen wird, wobei während der Trocknung zeitgleich eine hydrophile oder hydrophobe Kieselsäure zudosiert wird, so dass sich im finalen Produkt eine Konzentration an Kieselsäure von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen und wobei die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält.

Ganz besonders bevorzugt ist hierbei ein Verfahren zur Herstellung einer partikulären Biomasse ausgehend von einer Fermentationsbrühe, dadurch gekennzeichnet, dass die Fermentationsbrühe einer Düsensprühtrocknung oder einer Wirbelschichtgranulationstrocknung unterzogen wird, wobei während der Trocknung zeitgleich eine hydrophile Kieselsäure zudosiert wird, so dass sich im finalen Produkt eine Konzentration an hydrophiler Kieselsäure von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen, und wobei die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält und wobei die hydrophile Kieselsäure vorzugsweise eine spezifische Oberfläche (ISO 9277) von 130 bis 600 m²/g, vorzugsweise 160 bis 550 m²/g, und einen Dioctyladipat-Absorptionswert von 1,5-4,0 ml/g, vorzugsweise 2,0 - 3,2 ml/g, aufweist.

Ganz besonders bevorzugt ist hierbei ebenso ein Verfahren zur Herstellung einer partikulären Biomasse ausgehend von einer Fermentationsbrühe, dadurch gekennzeichnet, dass die Fermentationsbrühe einer Düsensprühtrocknung oder einer Wirbelschichtgranulationstrocknung unterzogen wird, wobei während der Trocknung zeitgleich eine hydrophobe Kieselsäure zudosiert wird, so dass sich im finalen Produkt eine Konzentration an hydrophober Kieselsäure von 0,1 bis 8,0 Gew.-%, insbesondere 0,2 bis 6,0 Gew.-%, vor allem 0,5 bis
2,5 Gew.-%, insbesondere 0,5 bis 1,5 Gew.-% oder 0,5 bis 1 Gew.-%, einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen, und wobei die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält und wobei die hydrophobe Kieselsäure vorzugsweise eine Methanolbenetzbarkeit von mindestens 40 %, vorzugsweise mindestens 45 %, besonders bevorzugt mindestens 50 %, insbesondere 40 bis 65 %, vor allem 50 bis 60 %, aufweist.

Besonders bevorzugt ist erfindungsgemäß auch ein Verfahren zur Herstellung einer partikulären Biomasse, die einen oxidationsempfindlichen Wertstoff enthält, ausgehend von einer Fermentationsbrühe, dadurch gekennzeichnet, dass die Fermentationsbrühe einer Düsensprühtrocknung oder einer Wirbelschichtgranulationstrocknung unterzogen wird, wobei nach der Trocknung in einem zusätzlichen Verfahrensschritt eine hydrophile oder hydrophobe Kieselsäure zudosiert wird, so dass sich im finalen Produkt eine Konzentration an Kieselsäure von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen, und wobei die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält.

Ganz besonders bevorzugt ist hierbei ein Verfahren zur Herstellung einer partikulären Biomasse ausgehend von einer Fermentationsbrühe, dadurch gekennzeichnet, dass die Fermentationsbrühe einer Düsensprühtrocknung oder einer Wirbelschichtgranulationstrocknung unterzogen wird, wobei nach der Trocknung in einem zusätzlichen Verfahrensschritt eine hydrophile Kieselsäure zudosiert wird, so dass sich im finalen Produkt eine Konzentration an hydrophiler Kieselsäure von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen, und wobei die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält und wobei die hydrophile Kieselsäure vorzugsweise eine spezifische Oberfläche (ISO 9277) von 130 bis 600 m²/g, vorzugsweise 160 bis 550 m²/g, und einen Dioctyladipat-Absorptionswert von 1,5-4,0 ml/g, vorzugsweise 2,0 - 3,2 ml/g, aufweist.

Ganz besonders bevorzugt ist hierbei ebenso ein Verfahren zur Herstellung einer partikulären Biomasse ausgehend von einer Fermentationsbrühe, dadurch gekennzeichnet, dass die Fermentationsbrühe einer Düsensprühtrocknung oder einer Wirbelschichtgranulationstrocknung unterzogen wird, wobei nach der Trocknung in einem zusätzlichen Verfahrensschritt eine hydrophobe Kieselsäure zudosiert wird, so dass sich im finalen Produkt eine Konzentration an hydrophober Kieselsäure von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen, und wobei die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält und wobei die hydrophobe Kieselsäure vorzugsweise eine Methanolbenetzbarkeit von mindestens 40 %, vorzugsweise mindestens 45 %, besonders bevorzugt mindestens 50 %, insbesondere 40 bis 65 %, vor allem 50 bis 60 %, aufweist.

Aber auch andere Trocknungsverfahren wie Trocknung im Fließbett und Trommeltrockner sowie die Sprühtrocknung unter Verwendung einer rotierenden Scheibe haben sich als sehr gut geeignet zur Trocknung der Fermentationsbrühe herausgestellt.

Die durch die thermische Trocknung erzeugten Partikel haben eine ausgezeichnete Festigkeit und weisen durch ihre im Wesentlichen runde Form bedingt sehr gute Schütt- und Fließeigenschaften auf. Die Partikel haben außerdem eine geringe Restfeuchte.

Durch die Trocknung wird ein rieselfähiges, feinteiliges oder grobkörniges Produkt, vorzugsweise Granulat, erhalten. Gegebenenfalls kann aus dem erhaltenen Granulat durch Sieben oder Staubabtrennung ein Produkt mit der gewünschten Korngröße erhalten werden.

Sofern ein rieselfähiges feinteiliges Pulver erhalten wurde, kann dieses gegebenenfalls durch geeignete Kompaktier- oder Granulierverfahren in ein grobkörniges, gut rieselfähiges, lagerbares und weitgehend staubfreies Produkt überführt werden.

Unter "rieselfähig" ist erfindungsgemäß ein Pulver zu verstehen, das aus einer Serie von Glasauslaufgefäßen mit verschieden großen Auslassöffnungen mindestens aus dem Gefäß mit der Öffnung 5 Millimeter ungehindert auslaufen kann (Klein: Seifen, Öle, Fette, Wachse 94, 12 (1968)).

Unter "feinteilig" ist erfindungsgemäß ein Pulver mit einem überwiegenden Anteil (> 50 %) einer Korngröße von 20 bis 100 Mikrometer Durchmesser zu verstehen.

Unter "grobkörnig" ist erfindungsgemäß ein Pulver mit einem überwiegenden Anteil (>50 %) einer Korngröße von 100 bis 2500 Mikrometer Durchmesser zu verstehen.

Unter "staubfrei" ist erfindungsgemäß ein Pulver zu verstehen, das lediglich geringe Anteile (< 10 %, vorzugsweise < 5 %) an Korngrößen unter 100 Mikrometer enthält.

Die Bestimmung der Korn- bzw. Teilchengrößen erfolgt erfindungsgemäß vorzugsweise durch Methoden der Laserbeugungsspektrometrie. Die anzuwendenden Methoden sind im Lehrbuch "Teilchengrößenmessung in der Laborpraxis" von R.H. Müller und R. Schuhmann, Wissenschaftliche Verlagsgesellschaft Stuttgart (1996) sowie im Lehrbuch "Introduction to Particle Technology" von M. Rhodes, Verlag Wiley & Sons (1998) beschrieben. Sofern unterschiedliche Methoden anwendbar sind, wird bevorzugt die zuerst genannte anwendbare Methode aus dem Lehrbuch von R.H. Müller und R. Schuhmann zur Teilchengrößenmessung angewendet.

Die durch erfindungsgemäße Trocknungsverfahren erhaltenen Produkte besitzen vorzugsweise einen Anteil von mindestens 80 Gew.-%, insbesondere mindestens 90 Gew.-%, besonders bevorzugt mindestens 95 Gew.-%, an Partikeln mit einer Korngröße von 100 bis 3500 Mikrometern, vorzugsweise 100 bis 3000 Mikrometern, vor allem 100 bis 2500 Mikrometern.

Die erfindungsgemäß erhaltenen Produkte eines Wirbelschichtgranulationsverfahrens besitzen hierbei vorzugsweise einen Anteil von mindestens 80 Gew.-%, insbesondere mindestens 90 Gew.-%, besonders bevorzugt mindestens 95 Gew.-%, an Partikeln mit einer Korngröße von 200 bis 3500 Mikrometern, vorzugsweise 300 bis 3000 Mikrometern, vor allem 500 bis 2500 Mikrometern.

Die erfindungsgemäß erhaltenen Produkte eines Düsensprühtrocknungsverfahrens besitzen hingegen vorzugsweise einen Anteil von mindestens 80 Gew.-%, insbesondere mindestens 90 Gew.-%, besonders bevorzugt mindestens 95 Gew.-%, an Partikeln mit einer Korngröße von 100 bis 500 Mikrometern, vorzugsweise 100 bis 400 Mikrometern, vor allem 100 bis 300 Mikrometern.

Aufgrund des Herstellverfahrens sind vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet. Vor allem die im Wesentlichen sphärische Ausbildung der Partikel bedingt die hervorragenden Schütt- und Fließeigenschaften des erfindungsgemäßen Produkts.

Unter "im Wesentlichen sphärisch ausgebildet" ist erfindungsgemäß zu verstehen, dass der Durchmesser eines Partikels in alle Raumrichtungen im Wesentlichen gleich ist. Unter "im Wesentlichen gleich" ist hierbei zu verstehen, dass die Abweichung des Durchmessers eines Partikels in zwei beliebige Raumrichtungen maximal 20 %, vorzugsweise maximal 15 %, insbesondere maximal 10 %, besonders bevorzugt maximal 5 %, beträgt.

Der Anteil an Staub, d.h. Teilchen mit einer Korngröße von kleiner 100 Mikrometern, liegt vorzugsweise bei maximal 10 Gew.-%, insbesondere maximal 8 Gew.-% besonders bevorzugt maximal 5 Gew.-%.

Das Schüttgewicht der erfindungsgemäßen Produkte liegt vorzugsweise bei 400 bis 800 kg/m³, besonders bevorzugt bei 450 bis 700 kg/m³.

Die in der partikulären Biomasse enthaltene hydrophile Kieselsäure weist vorzugsweise die zuvor genannten Eigenschaften auf, also insbesondere eine spezifische Oberfläche von 130 bis 600 m²/g, vorzugsweise 160 bis 550 m²/g, und einen Dioctyladipat-Absorptionswert von 1,5 - 4,0 ml/g, vorzugsweise 2,0 - 3,2 ml/g.

Die in der partikulären Biomasse enthaltene hydrophobe Kieselsäure weist vorzugsweise ebenfalls die zuvor genannten Eigenschaften auf, also insbesondere eine Methanolbenetzbarkeit von mindestens 40 %, vorzugsweise mindestens 45 %, besonders bevorzugt mindestens 50 %, insbesondere 40 bis 65 %, vor allem 50 bis 60 %.

Weiterhin weist die erfindungsgemäße partikuläre Biomasse vorzugsweise mindestens eine weitere der zuvor genannten Eigenschaften auf, insbesondere hinsichtlich Partikelgröße und Partikelverteilung sowie vorzugsweise auch hinsichtlich der Partikelform.

So zeichnet sich die partikuläre Biomasse vorzugsweise dadurch aus, dass mindestens 80 Gew.-%, insbesondere mindestens 90 Gew.-%, vorzugsweise mindestens 95 Gew.-%, der Partikel eine Korngröße von 100 bis 3500 Mikrometern, vorzugsweise 100 bis 2500 Mikrometern, aufweisen und die partikuläre Biomasse vorzugsweise rieselfähig und staubarm ist.

Weiterhin zeichnet sie sich vorzugsweise dadurch aus, dass mindestens 50 Gew.-%, insbesondere mindestens 70 Gew.-%, vorzugsweise mindestens 90 Gew.-%, der Partikel - im Sinne der zuvor gegebenen Definition - im Wesentlichen sphärisch ausgebildet sind.

Hinsichtlich bevorzugter Natur der Biomasse bzw. der in der Biomasse enthaltenen Zellen sowie hinsichtlich bevorzugt enthaltenem Wertstoff wird auf das zuvor Ausgeführte verwiesen. Bei dem oxidationsempfindlichen Wertstoff handelt es sich besonders bevorzugt um eine omega-3-Fettsäure oder omega-6-Fettsäure. Die partikuläre Biomasse umfasst vorzugsweise Zellen des Taxons Labyrinthulomycetes, insbesondere solche der Familie der Thraustochytriaceae, besonders bevorzugt der Gattungen Thraustochytrium, Schizochytrium oder Aurantiochytrium, vor allem solche der Gattung Aurantiochytrium.

Besonders bevorzugter Gegenstand der vorliegenden Erfindung ist hierbei eine partikuläre rieselfähige Biomasse, die hydrophile und/oder hydrophobe Kieselsäure, in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikel eine Korngröße von 100 bis 3500 Mikrometern, vorzugsweise 100 bis
3000 Mikrometern, vor allem 100 bis 2500 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Ganz besonders bevorzugt ist hierbei eine partikuläre rieselfähige Biomasse, die hydrophile und/oder hydrophobe Kieselsäure, in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikeln eine Korngröße von 200 bis 3500 Mikrometern, vorzugsweise 300 bis 3000 Mikrometern, vor allem 500 bis 2500 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Ganz besonders bevorzugt ist hierbei ebenso eine partikuläre rieselfähige Biomasse, die hydrophile und/oder hydrophobe Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikeln eine Korngröße von 100 bis 500 Mikrometern, vorzugsweise 100 bis 400 Mikrometern, vor allem 100 bis 300 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Besonders bevorzugter Gegenstand der vorliegenden Erfindung ist hierbei insbesondere eine partikuläre rieselfähige Biomasse, die hydrophile Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikel eine Korngröße von 100 bis 3500 Mikrometern, vorzugsweise 100 bis 3000 Mikrometern, vor allem 100 bis 2500 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Ganz besonders bevorzugt ist hierbei eine partikuläre rieselfähige Biomasse, die hydrophile Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikeln eine Korngröße von 200 bis 3500 Mikrometern, vorzugsweise 300 bis 3000 Mikrometern, vor allem 500 bis 2500 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Ganz besonders bevorzugt ist hierbei ebenso eine partikuläre rieselfähige Biomasse, die hydrophile Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des
Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält wobei die Zellen der Biomasse einen Lipidgehalt von mindestens 20 Gew.-%, bezogen auf die Zelltrockenmasse, aufweisen und wobei mindestens

80 Gew.-%, insbesondere mindestens 90 Gew.-%, bevorzugt mindestens 95 Gew.-%, der enthaltenen Partikeln eine Korngröße von 100 bis 500 Mikrometern, vorzugsweise 100 bis 400 Mikrometern, vor allem 100 bis 300 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Besonders bevorzugter Gegenstand der vorliegenden Erfindung ist hierbei insbesondere auch eine partikuläre rieselfähige Biomasse, die hydrophobe Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikel eine Korngröße von 100 bis 3500 Mikrometern, vorzugsweise 100 bis 3000 Mikrometern, vor allem 100 bis 2500 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Ganz besonders bevorzugt ist hierbei eine partikuläre rieselfähige Biomasse, die hydrophobe Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikeln eine Korngröße von 200 bis 3500 Mikrometern, vorzugsweise 300 bis 3000 Mikrometern, vor allem 500 bis 2500 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Ganz besonders bevorzugt ist hierbei ebenso eine partikuläre rieselfähige Biomasse, die hydrophobe Kieselsäure in einer Menge von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, umfasst sowie Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium, enthält, wobei die Zellen der Biomasse einen Lipidgehalt von mindestens 20 Gew.-%, bezogen auf die Zelltrockenmasse, aufweisen und wobei mindestens 80 Gew.-%, insbesondere mindestens 90 Gew.-%, bevorzugt mindestens 95 Gew.-%, der enthaltenen Partikeln eine Korngröße von 100 bis 500 Mikrometern, vorzugsweise 100 bis 400 Mikrometern, vor allem 100 bis 300 Mikrometern, aufweisen, und wobei - gemäß obiger Definition - vorzugsweise mindestens 50 oder 60 Gew.-%, insbesondere mindestens 70 oder 80 Gew.-%, besonders bevorzugt mindestens 90 oder 95 Gew.-%, vor allem im Wesentlichen alle Partikel im Wesentlichen sphärisch ausgebildet.

Die erfindungsgemäß erhaltene getrocknete partikuläre Biomasse kann auf unterschiedliche Weise verwendet werden. Nach der erfindungsgemäßen Trocknung der Biomasse erfolgt vorzugsweise eine Lagerung bzw. Verpackung der getrockneten Biomasse. Anschließend kann die Biomasse beispielsweise vor Ort eingesetzt werden, um ein Futter- oder Nahrungsmittel herzustellen oder um den Wertstoff aus der Biomasse zu isolieren.

Ein Futter- oder Nahrungsmittel enthaltend eine erfindungsgemäße partikuläre Biomasse ist daher ein weiterer Gegenstand der vorliegenden Erfindung.

Um die Bioverfügbarkeit in dem herzustellenden Futter- oder Nahrungsmittel zu erhöhen und/oder um die Isolierung des in der Biomasse enthaltenen Wertstoffs zu erleichtern, wird die partikuläre Biomasse - unmittelbar vor der Herstellung des Futter- oder Nahrungsmittels - vorzugsweise einem Zellaufschlussverfahren unterzogen.

Vor der Durchführung des Zellaufschlussverfahrens wird vorzugsweise ausgehend von der getrockneten Biomasse eine Zellsuspension hergestellt. Hierzu wird die getrocknete Biomasse mit Wasser oder einer wässrigen Lösung vermischt, um eine Zellsuspension mit einem Feuchtigkeitsgehalt von vorzugsweise mindestens 30 Gew.-%, insbesondere 30 bis 90 Gew.-%, besonders bevorzugt 40 bis 80 Gew.-%, vor allem 50 bis 75 Gew.-%, herzustellen.

Der Zellaufschluss kann anschließend unter Einsatz der dem Fachmann bekannten Zellaufschlussverfahren erfolgen, wie beispielsweise durch einen Schneckenextruder, eine Schlägermühle, eine Luftdüsenmühle oder durch Anwendung erhöhten Drucks, beispielsweise durch das sogenannte French-Press-Verfahren. Alternativ oder zusätzlich kann der Zellaufschluss durch den Einsatz von zellwandverdauenden Enzymen erfolgen.

Der Zellaufschluss wird erfindungsgemäß vorzugsweise unter Verwendung eines Rotor-Stator-Systems durchgeführt. Das Rotor-Stator-System basiert auf einem stationären Teil, der Stator genannt wird, und einem rotierenden Teil, dem Rotor. Der Rotor besitzt typischerweise eine Umfangsgeschwindigkeit von mindestens 5 m/s, beispielsweise 10 bis 30 m/s, die Spaltbreite zwischen Rotor und Stator kann beispielsweise 0,1 - 0,5 mm betragen. Zur Durchführung des Zellaufschlusses wird die Zell-Suspension in den Zwischenraum zwischen Stator und Rotor zugegeben. In diesem Spalt erfahren die Zellen eine Scherbeanspruchung und zusätzlich entstehen Turbulenzen. Diese beiden Faktoren bewirken den Zellaufschluss.

In einer bevorzugten Ausführungsform erfolgt die Herstellung der Suspension im Rotor-Stator-System unter Verwendung eines feststoffmischenden Aufsatzes. Unter einem feststoffmischenden Aufsatz ist hierbei eine Vorrichtung zu verstehen, die das separate Einbringen von einerseits Feststoff und andererseits Wasser bzw. wässriger Lösung in das Rotor-Stator-System erlaubt. Die Suspension wird somit erst während des Zellaufschlusses bzw. unmittelbar vor dem Zellaufschluss durch Vermischung in dem feststoffmischenden Aufsatz hergestellt. Es wurde gefunden, dass unter Verwendung eines solchen feststoffmischenden Aufsatzes Suspensionen mit sehr hohen Feststoffgehalten dem Zellaufschluss unterzogen werden können, was mit Hinblick auf die nachfolgende Verarbeitung besonders vorteilhaft ist. Suspensionen, die unter Verwendung eines feststoffmischenden Aufsatzes in dem Rotor-Stator-System eingesetzt werden, weisen vorzugsweise einen Feststoffgehalt von 20-50 Gew.-%, besonders bevorzugt von 30-50 Gew.-%, auf.

Falls zur Herstellung der Zellsuspension eine wässrige Lösung eingesetzt wird, dann kann diese insbesondere weitere Nahrungsmittelkomponenten - wie etwa Vitamine oder Salze - enthalten.

Der Energieeintrag auf die Zellen, insbesondere unter Verwendung eines Rotor-Stator-Systems, beträgt erfindungsgemäß vorzugsweise maximal 50 kWh pro Tonne Suspension, insbesondere maximal 40, 35 oder 30 kWh pro Tonne Suspension, besonders bevorzugt maximal 25, 20 oder 15 kWh pro Tonne Suspension. Bevorzugte Bereiche stellen hierbei Energieeinträge von 0,1 - 50 kWh pro Tonne Suspension, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, dar.

Die "Zellaufschlussrate" des erfindungsgemäßen Verfahrens beträgt vorzugsweise mindestens 50 %, besonders bevorzugt mindestens 60, 70 oder 80 %, vor allem mindestens 85, 90 oder 95 %. Unter der "Zellaufschlussrate" ist die Anzahl der aufgeschlossenen Zellen nach Beendigung des Zellaufschlussverfahrens im Verhältnis zur Gesamtzahl der Zellen zu verstehen. Die Zellaufschlussrate kann beispielsweise visuell unter Verwendung eines Mikroskops bestimmt werden als das Verhältnis der Anzahl aufgeschlossener Zellen in Bezug zur Gesamtzahl der Zellen.

Um die Wertstoffe, insbesondere Lipide, gegen oxidativen Abbau zu stabilisieren, können in der Zellsuspension, die für den Zellaufschluss verwendet wird, zusätzlich Antioxidantien enthalten sein. Bevorzugte Antioxidantien stellen hierbei BHT, BHA, TBHA, Ethoxyquin, beta-Carotin, Vitamin E und Vitamin C dar. Das Antioxidans ist, sofern eingesetzt, vorzugsweise in einer Menge von 0,01 bis 2 Gew.-% enthalten. Die Antioxidantien werden hierbei in einer bevorzugten Ausführungsform bereits nach Beendigung der Fermentation dem Fermentationsmedium hinzugegeben.

Sowohl die intakte als auch die aufgeschlossene Biomasse können zur Herstellung eines Nahrungs- oder Futtermittels verwendet werden, bei welchem die Biomasse bzw. der Wertstoff vorzugsweise mit anderen Nahrungs- oder Futtermittelinhaltsstoffen vermischt werden und anschließend zu dem Nahrungs- oder Futtermittel verarbeitet werden.

Die Verarbeitung des Gemisches aus Biomasse und weiteren Nahrungs- oder Futtermittelinhaltsstoffen erfolgt in einer bevorzugten Ausführungsform durch ein Extrusionsverfahren, um verkaufsfertige Portionen des Nahrungs- oder Futtermittels zu erhalten. Alternativ kann beispielsweise auch ein Pelletierverfahren eingesetzt werden.

Im Extrusionsverfahren wird vorzugsweise ein Schnecken- oder Doppelschneckenextruder eingesetzt. Das Extrusionsverfahren wird vorzugsweise bei einer Temperatur von 80 - 220° C, insbesondere 100 - 190 °C, einem Druck von 10 - 40 Bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Bei einer erfindungsgemäß bevorzugten Art des Extrusionsverfahrens umfasst das Verfahren einen Kompaktierungs- und einen Kompressionsschritt.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken.

Die weiteren Nahrungs- oder Futtermittelinhaltsstoffe werden vor dem Vermischen mit den aufgeschlossenen Zellen - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

Ein erfindungsgemäß bevorzugtes Verfahren zur Herstellung eines Nahrungs- oder Futtermittels umfasst daher die folgenden Schritte:
a) Herstellung einer Biomasse durch Fermentierung von Pilzen oder Mikroalgen, die einen Wertstoff, vorzugsweise ein Lipid, besonders bevorzugt omega-3-Fettsäuren, produzieren;
b) schonende thermische Trocknung eines Gemisches aus erhaltener Biomasse und hydrophiler oder hydrophober Kieselsäure, vorzugsweise durch Düsensprühtrocknung oder Wirbelschichtgranulation, zu einer partikulären Zusammensetzung, wobei die Kieselsäure vorzugsweise in einer Menge hinzugegeben wird, dass sich eine finale Konzentration von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, an Kieselsäure im finalen Produkt einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen und wobei die hydrophile Kieselsäure vorzugsweise eine spezifische Oberfläche (ISO 9277) von 130 bis 600 m²/g, insbesondere 160 bis 550 m²/g, und einen Dioctyladipat-Absorptionswert von 1,5 - 4,0 ml/g, vorzugsweise 2,0 - 3,2 ml/g, aufweist und die hydrophobe Kieselsäure vorzugsweise eine Methanolbenetzbarkeit von mindestens 40 %, vorzugsweise mindestens 45 %, besonders bevorzugt mindestens 50 %, insbesondere 40 bis 65 %, vor allem 50 bis 60 %, aufweist;
c) Vermischung der Biomasse und/oder daraus isolierter Wertstoffe, gegebenenfalls nach vorheriger Durchführung eines Zellaufschlussverfahrens, mit weiteren Nahrungs- oder Futtermittelinhaltsstoffen;
d) Herstellung des finalen Produkts durch ein Kompaktierungs- oder Extrusionsverfahren.

Ein erfindungsgemäß ganz besonders bevorzugtes Verfahren zur Herstellung eines Nahrungs- oder Futtermittels umfasst hierbei die folgenden Schritte:
a) Herstellung einer Biomasse durch Fermentierung von Zellen des Taxons Labyrinthulomycetes, insbesondere solche der Familie der Thraustochytriaceae, vorzugsweise solche der Gattungen Thraustochytrium, Schizochytrium oder Aurantiochytrium, vor allem solche der Gattung Aurantiochytrium;
b) schonende thermische Trocknung eines Gemisches aus erhaltener Biomasse und hydrophiler oder hydrophober Kieselsäure, vorzugsweise durch Düsensprühtrocknung oder Wirbelschichtgranulation, zu einer partikulären Zusammensetzung mit einem Feuchtigkeitsgehalt von weniger als 15 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%, wobei die Kieselsäure vorzugsweise in einer Menge hinzugegeben wird, dass sich eine finale Konzentration von 1 bis 8 Gew.-%, vorzugsweise 2 bis 6 Gew.-%, an Kieselsäure im finalen Produkt einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen und wobei die Kieselsäure vorzugsweise eine spezifische Oberfläche (ISO 9277) von 130 bis 600 m²/g, insbesondere 160 bis 550 m²/g, und einen Dioctyladipat-Absorptionswert von 1,5 - 4,0 ml/g, vorzugsweise 2,0 - 3,2 ml/g, aufweist und die hydrophobe Kieselsäure vorzugsweise eine Methanolbenetzbarkeit von mindestens 40 %, vorzugsweise mindestens 45 %, besonders bevorzugt mindestens 50 %, insbesondere 40 bis 65 %, vor allem 50 bis 60 %, aufweist;
c) Zellaufschluss, vorzugsweise unter Anwendung eines Energieeintrags von maximal 50 kWh pro Tonne Suspension, vorzugsweise von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, vorzugsweise unter Verwendung eines Rotor-Stator-Systems;
d) Vermischung der aufgeschlossenen Zellen und/oder daraus isolierter Wertstoffe mit weiteren Nahrungs- oder Futtermittelinhaltsstoffen;
e) Herstellung des finalen Produkts durch ein Kompaktierungs- oder Extrusionsverfahren.

Ein weiteres erfindungsgemäß bevorzugtes Verfahren zur Herstellung eines Nahrungs- oder Futtermittels umfasst die folgenden Schritte:
a) Herstellung einer Biomasse durch Fermentierung von Zellen des Taxons Labyrinthulomycetes, insbesondere solchen der Familie der Thraustochytriaceae, vorzugsweise solchen der Gattungen Thraustochytrium, Schizochytrium oder Aurantiochytrium, vor allem solche der Gattung Aurantiochytrium,;
b) schonende thermische Trocknung der erhaltenen Biomasse, vorzugsweise durch Düsensprühtrocknung oder Wirbelschichtgranulation, zu einer partikulären Zusammensetzung,
c) Vermischung der getrockneten Biomasse mit einer hydrophilen oder hydrophoben Kieselsäure, wobei die Kieselsäure vorzugsweise in einer Menge hinzugegeben wird, dass sich eine finale Konzentration von 0,1 bis 8,0 Gew.-%, insbesondere 0,2 bis 6,0 Gew.-%, vor allem 0,5 bis 2,5 Gew.-%, insbesondere 0,5 bis 1,5 Gew.-% oder 0,5 bis 1 Gew.-%, an Kieselsäure im finalen Produkt einstellt, wobei das Produkt einen Zellgehalt von mindestens 60 Gew.-% aufweist und wobei die Zellen der Biomasse einen Lipidgehalt von mindestens 20 Gew.-%, bezogen auf die Zelltrockenmasse, aufweisen und wobei die hydrophile Kieselsäure vorzugsweise eine spezifische Oberfläche (ISO 9277) von 130 bis 600 m²/g, insbesondere 160 bis 550 m²/g, und einen Dioctyladipat-Absorptionswert von 1,5 - 4,0 ml/g, vorzugsweise 2,0 - 3,2 ml/g, aufweist und die hydrophobe Kieselsäure vorzugsweise eine Methanolbenetzbarkeit von mindestens 40 %, vorzugsweise mindestens 45 %, besonders bevorzugt mindestens 50 %, insbesondere 40 bis 65 %, vor allem 50 bis 60 %, aufweist;
c) Vermischung der Biomasse und/oder daraus isolierter Wertstoffe, gegebenenfalls nach vorheriger Durchführung eines Zellaufschlussverfahrens, mit weiteren Nahrungs- oder Futtermittelinhaltsstoffen;
d) Herstellung des finalen Produkts durch ein Kompaktierungs- oder Extrusionsverfahren.

Erfindungsgemäß bevorzugte Verfahren zur Herstellung eines Nahrungs- oder Futtermittels zeichnen sich vorzugsweise dadurch aus, dass in keinem Verfahrensschritt ein höherer Energieeintrag auf die Biomasse erfolgt als 50 kWh pro Tonne Suspension. Vorzugsweise beträgt der Energieeintrag auf die Biomasse maximal 40 oder 35 kWh, insbesondere maximal 30 oder 25 kWh, besonders bevorzugt 20 oder 15 kWh, jeweils pro Tonne Suspension. Auf diese Weise wird zusätzlich sichergestellt, dass der enthaltene Wertstoff möglichst wenig geschädigt wird.

Die aufgeschlossenen Zellen machen vorzugsweise 0,5-20 Gew.-%, insbesondere 1-10 Gew.-%, vorzugsweise 2-8 Gew.-% des Nahrungs- oder Futtermittels bzw. der zur Herstellung des Nahrungs- oder Futtermittels eingesetzten Zusammensetzung aus. Bei dem Nahrungs- oder Futtermittel handelt es sich vorzugsweise um ein Mittel zum Einsatz in der Aquakultur oder um ein Nahrungs- oder Futtermittel zum Einsatz in der Geflügelzucht, Schweinezucht oder Rinderzucht. Bei dem Futtermittel kann es sich auch um ein Futtermittel handeln, das eingesetzt wird, um Kleinlebewesen zu züchten, die in der Aquakultur als Futtermittel eingesetzt werden können. Bei den Kleinlebewesen kann es sich beispielsweise um Nematoden, Crustaceen oder Rotiferen handeln. Das Futtermittel liegt vorzugsweise flockenförmig, kugelförmig oder tablettenförmig vor. Ein durch Extrusion erhältliches Futtermittel hat vorzugsweise einen Feuchtigkeitsgehalt von weniger als 5 Gew.-%, besonders bevorzugt von 0,2 bis 4 Gew.-%.

Die anderen Nahrungs- oder Futtermittelinhaltsstoffe sind vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

Als proteinhaltige Komponente, die zusätzlich Fette enthält, können beispielsweise eingesetzt werden Fischmehl, Krillmehl, Muschelmehl, Kalmarmehl oder Schrimpsschalen. Als fetthaltige Komponente kann alternativ auch Fischöl eingesetzt werden. Als fetthaltige Komponente kann auch ein Pflanzenöl eingesetzt werden, insbesondere Öl aus Sojabohnen, Rapssamen, Sonnenblumenkernen und Flachssamen. Als kohlenhydrathaltige Komponente kann beispielsweise Weizenmehl, Sonnenblumenmehl, Sojablumenmehl oder Getreidegluten eingesetzt werden.

Der Gesamtgehalt an Öl in dem Futtermittel - inklusive des Öls aus den öl-haltigen Zellen - beträgt vorzugsweise 15-50 Gew.-%.

Das Futtermittel zum Einsatz in der Aquakultur wird vorzugsweise verwendet um Flossenfische und Crustaceen zu züchten, die vorzugsweise der Ernährung von Menschen dienen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich um ein Futtermittel für die Lachs-Zucht. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Alternativ kann es sich auch um ein Futtermittel handeln, das zur Anzucht von Fischen dient, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau. Das so erhaltene Fischmehl oder Fischöl kann wiederum in der Aquakultur zur Zucht von Speisefischen bzw. Crustaceen eingesetzt werden.

Die Aquakultur kann in Teichen, Tanks, Becken oder auch in abgegrenzten Arealen im Meer oder in Seen, hierbei insbesondere in Käfigen oder Netzpferchen, erfolgen. Die Aquakultur kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

### Ausführungsbeispiele

### Beispiel 1: Herstellung einer DHA-enthaltenden partikulären rieselfähigen Biomasse, die eine hydrophile Kieselsäure enthält

Zur Produktion von DHA wurde der Stamm Schizochytrium limacinum SR21 eingesetzt. Dieser ist hinterlegt beim NIBH unter FERM BP-5034 sowie beim IFO unter IFO 32693. Der Stamm S. limacinum SR21 wurde ursprünglich aus Meerwasser isoliert (Nakahara et al. 1996, JAOCS, 73(10); Honda Mycol. Res. 1998).

Die Fermentation des Stammes erfolgte in einem Medium, das 50 % künstliches Meerwasser (Sigma Aldrich) enthielt und des Weiteren folgende Komponenten enthielt: 60 g/l Glucose, 0,7 g/l Maisquellwasser (Sigma Aldrich), 2 g/l (NH₄)₂SO₄ und 3 g/l KH₂PO₄.

Die Fermentation erfolgte bei 28°C, einem pH-Wert von 4,0, einer Belüftungsrate von 0,5 vvm und einer Rührung von 200 rpm für 60 Stunden. Nach Beendigung der Fermentation wurde der Fermentationsbrühe ein Antioxidans zugegeben und die Fermentationsbrühe danach mindestens 20 Minuten auf 60°C erhitzt.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Während der Sprühtrocknung wurde in mehreren Vergleichsversuchen gleichzeitig durch eine zweite Düse hydrophile Kieselsäure (Sipernat® 22S, Evonik, Deutschland) in trockener Form in einer Menge zudosiert, so dass sich im finalen Produkt eine Konzentration von 0,5; 1,0 oder 1,5 Gew.-% an hydrophiler Kieselsäure einstellte. Durch Sprühtrocknung wurde so durch Zugabe einer relativ geringen Menge an Zuschlagmittel jeweils ein rieselfähiges Pulver mit einer Trockenmasse von mehr als 95 Gew.-% erhalten.

Im Vergleichsversuch ohne Zugabe der hydrophilen Kieselsäure wurde kein rieselfähiges Pulver erhalten

### Beispiel 2: Herstellung einer DHA-enthaltenden partikulären rieselfähigen Biomasse, die eine hydrophobe Kieselsäure enthält

Zur Produktion von DHA wurde der Stamm Schizochytrium limacinum SR21 eingesetzt. Dieser ist hinterlegt beim NIBH unter FERM BP-5034 sowie beim IFO unter IFO 32693. Der Stamm S. limacinum SR21 wurde ursprünglich aus Meerwasser isoliert (Nakahara et al. 1996, JAOCS, 73(10); Honda Mycol. Res. 1998).

Die Fermentation des Stammes erfolgte in einem Medium, das 50 % künstliches Meerwasser (Sigma Aldrich) enthielt und des Weiteren folgende Komponenten enthielt: 60 g/l Glucose, 0,7 g/l Maisquellwasser (Sigma Aldrich), 2 g/l (NH₄)₂SO₄ und 3 g/l KH₂PO₄.

Die Fermentation erfolgte bei 28°C, einem pH-Wert von 4,0, einer Belüftungsrate von 0,5 vvm und einer Rührung von 200 rpm für 60 Stunden. Nach Beendigung der Fermentation wurde der Fermentationsbrühe ein Antioxidans zugegeben und die Fermentationsbrühe danach mindestens 20 Minuten auf 60°C erhitzt.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Während der Sprühtrocknung wurde in mehreren Vergleichsversuchen gleichzeitig durch eine zweite Düse hydrophobe Kieselsäure (Sipernat® D10, Evonik, Deutschland) in trockener Form in einer Menge zudosiert, so dass sich im finalen Produkt eine Konzentration von 0,5; 1,0 oder 1,5 Gew.-% an hydrophiler Kieselsäure einstellte. Durch Sprühtrocknung wurde so durch Zugabe einer relativ geringen Menge an Zuschlagmittel ein rieselfähiges Pulver mit einer Trockenmasse von mehr als 95 Gew.-% erhalten.

Ohne Zugabe der hydrophoben Kieselsäure wurde kein rieselfähiges Pulver erhalten.

### Beispiel 3: Herstellung einer DHA-enthaltenden partikulären rieselfähigen Biomasse, die eine hydrophile Kieselsäure enthält

Zur Produktion von DHA wurde der Stamm Schizochytrium limacinum SR21 eingesetzt. Dieser ist hinterlegt beim NIBH unter FERM BP-5034 sowie beim IFO unter IFO 32693. Der Stamm S. limacinum SR21 wurde ursprünglich aus Meerwasser isoliert (Nakahara et al. 1996, JAOCS, 73(10); Honda Mycol. Res. 1998).

Die Fermentation des Stammes erfolgte in einem Medium, das 50 % künstliches Meerwasser (Sigma Aldrich) enthielt und des Weiteren folgende Komponenten enthielt: 60 g/l Glucose, 0,7 g/l Maisquellwasser (Sigma Aldrich), 2 g/l (NH₄)₂SO₄ und 3 g/l KH₂PO₄.

Die Fermentation erfolgte bei 28°C, einem pH-Wert von 4,0, einer Belüftungsrate von 0,5 vvm und einer Rührung von 200 rpm für 60 Stunden. Nach Beendigung der Fermentation wurde der Fermentationsbrühe ein Antioxidans zugegeben und die Fermentationsbrühe danach mindestens 20 Minuten auf 60°C erhitzt.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Durch Sprühtrocknung wurde so ein nicht rieselfähiges Pulver mit einer Trockenmasse von mehr als 95 Gew.-% erhalten.

Das erhaltene Pulver wurde anschließend unter Verwendung eines kontinuierlichen Mischers (Gericke Kontimischer GCM, Gericke, Deutschland) in mehreren Vergleichsversuchen mit hydrophiler Kieselsäure (Sipernat® 22S) in einer Menge vermischt, so dass sich im finalen Produkt eine Konzentration von 0,5; 1,0 oder 1,5 Gew.-% an hydrophiler Kieselsäure einstellte. Auf diese Weise konnte die getrocknete Biomasse in eine rieselfähige Form überführt werden.

### Beispiel 4: Herstellung einer DHA-enthaltenden partikulären rieselfähigen Biomasse, die eine hydrophobe Kieselsäure enthält

Zur Produktion von DHA wurde der Stamm Schizochytrium limacinum SR21 eingesetzt. Dieser ist hinterlegt beim NIBH unter FERM BP-5034 sowie beim IFO unter IFO 32693. Der Stamm S. limacinum SR21 wurde ursprünglich aus Meerwasser isoliert (Nakahara et al. 1996, JAOCS, 73(10); Honda Mycol. Res. 1998).

Die Fermentation des Stammes erfolgte in einem Medium, das 50 % künstliches Meerwasser (Sigma Aldrich) enthielt und des Weiteren folgende Komponenten enthielt: 60 g/l Glucose, 0,7 g/l Maisquellwasser (Sigma Aldrich), 2 g/l (NH₄)₂SO₄ und 3 g/l KH₂PO₄.

Die Fermentation erfolgte bei 28°C, einem pH-Wert von 4,0, einer Belüftungsrate von 0,5 vvm und einer Rührung von 200 rpm für 60 Stunden. Nach Beendigung der Fermentation wurde der Fermentationsbrühe ein Antioxidans zugegeben und die Fermentationsbrühe danach mindestens 20 Minuten auf 60°C erhitzt.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Durch Sprühtrocknung wurde so ein nicht rieselfähiges Pulver mit einer Trockenmasse von mehr als 95 Gew.-% erhalten.

Das erhaltene Pulver wurde anschließend unter Verwendung eines kontinuierlichen Mischers (Gericke Kontimischer GCM, Gericke, Deutschland) in mehreren Vergleichsversuchen mit hydrophober Kieselsäure (Sipernat® D10, Evonik, Deutschland) in einer Menge vermischt, so dass sich im finalen Produkt eine Konzentration von 0,5; 1,0 oder 1,5 Gew.-% an hydrophober Kieselsäure einstellte. Auf diese Weise konnte die getrocknete Biomasse in eine rieselfähige Form überführt werden.

## Patentansprüche

1. Verfahren zur Herstellung einer partikulären rieselfähigen Biomasse, die einen oxidationsempfindlichen Wertstoff enthält, **dadurch gekennzeichnet, dass** zur Herstellung der partikulären Biomasse eine hydrophile oder hydrophobe Kieselsäure in einer Menge eingesetzt wird, so dass sich in der partikulären Biomasse eine Konzentration an hydrophiler oder hydrophober Kieselsäure von 0,1 bis 8 Gew.-% einstellt, wobei das Produkt einen Zellgehalt von mindestens 60 Gew.-% aufweist und wobei die Zellen der Biomasse einen Lipid-Gehalt von mindestens 20 Gew.-%, bezogen auf die Zelltrockenmasse, aufweisen.

2. Verfahren nach Anspruch 1, **dadurch gekennzeichnet, dass** die hydrophile Kieselsäure eine spezifische Oberfläche von 150 bis 600 m²/g und einen Dioctyladipat-Absorptionswert von 2 - 3,5 ml/g aufweist.

3. Verfahren gemäß Anspruch 1, **dadurch gekennzeichnet, dass** die hydrophobe Kieselsäure eine Methanolbenetzbarkeit von mindestens 40 %, vorzugsweise mindestens 45 %, besonders bevorzugt mindestens 50 %, aufweist.

4. Verfahren nach einem der vorhergehenden Ansprüche, **dadurch gekennzeichnet, dass** es sich bei dem oxidationsempfindlichen Wertstoff und eine ungesättigte Fettsäure, vorzugsweise eine omega-3-Fettsäure oder omega-6-Fettsäure, besonders bevorzugt DHA, handelt.

5. Verfahren nach einem der vorhergehenden Ansprüche, **dadurch gekennzeichnet, dass** die hydrophile oder hydrophobe Kieselsäure in einer Menge eingesetzt wird, so dass sich in der partikulären Biomasse eine Konzentration an Kieselsäure von 0,2 bis 6 Gew.-%, bevorzugt 0,5 bis 2,5 Gew.-%, einstellt.

6. Verfahren nach einem der vorhergehenden Ansprüche, **dadurch gekennzeichnet, dass** die Biomasse Zellen des Taxons Labyrinthulomycetes, insbesondere solche der Familie der Thraustochytriaceae, vorzugsweise solche der Gattungen Thraustochytrium, Schizochytrium oder Aurantiochytrium, besonders bevorzugt solche der Gattung Aurantiochytrium, enthält.

7. Verfahren nach einem der vorhergehenden Ansprüche, **dadurch gekennzeichnet, dass** die Biomasse zwecks Herstellung der partikulären Zusammensetzung in Form von Fermentationsbrühe mit einem Feststoffgehalt von 10 bis 50 Gew.-%, vorzugsweise 20 bis 40 Gew.-%, eingesetzt wird, und durch Trocknung in die partikuläre Biomasse überführt wird.

8. Verfahren nach einem der vorhergehenden Ansprüche, **dadurch gekennzeichnet, dass** die Trocknung der Biomasse durch ein thermisches Verfahren, vorzugsweise durch Düsensprühtrocknung oder Sprühgranulation, erfolgt, und die Kieselsäure vorzugsweise während des Trocknungsverfahrens zudosiert wird.

9. Partikuläre rieselfähige Biomasse, die einen oxidationsempfindlichen Wertstoff enthält, **dadurch gekennzeichnet, dass** sie einen Zellgehalt von mindestens 60 Gew.-% aufweist und hydrophile oder hydrophobe Kieselsäure in einer Menge von 0,1 bis 8 Gew.-%, vorzugsweise 0,2 bis 6 Gew.-%, besonders bevorzugt 0,5 bis 2,5 Gew.-%, enthält, wobei die Zellen der Biomasse einen Lipid-Gehalt von mindestens 20 Gew.-%, bezogen auf die Zelltrockenmasse, aufweisen und wobei die Biomasse Zellen des Taxons Labyrinthulomycetes umfasst.

10. Partikuläre Biomasse nach einem der vorhergehenden Ansprüche, **dadurch gekennzeichnet, dass** die hydrophile Kieselsäure eine spezifische Oberfläche von 130 bis 600 m2/g, vorzugsweise 160 bis 550 m2/g, und einen Dioctyladipat-Absorptionswert von 1,5 - 4,0 ml/g, vorzugsweise 2,0 - 3,2 ml/g, aufweist.

11. Partikuläre Biomasse nach Anspruch 9, **dadurch gekennzeichnet, dass** die hydrophobe Kieselsäure eine Methanolbenetzbarkeit von mindestens 40 %, vorzugsweise mindestens 45 %, besonders bevorzugt mindestens 50 %, aufweist.

12. Partikuläre Biomasse nach einem der vorhergehenden Ansprüche, **dadurch gekennzeichnet, dass** mindestens 80 Gew.-%, insbesondere mindestens 90 Gew.-%, vorzugsweise mindestens 95 Gew.-%, der Partikel eine Korngröße von 100 bis 3500 Mikrometern, vorzugsweise 100 bis 2500 Mikrometern, aufweisen und die partikuläre Biomasse vorzugsweise rieselfähig und staubarm ist.

13. Partikuläre Biomasse nach einem der vorhergehenden Ansprüche, **dadurch gekennzeichnet, dass** die Biomasse Zellen der Familie der Thraustochytriaceae, besonders bevorzugt der Gattungen Thraustochytrium, Schizochytrium oder Ulkenia, enthält.

14. Futter- oder Nahrungsmittel enthaltend eine partikuläre Biomasse nach einem der vorhergehenden Ansprüche sowie weitere Futtermittelinhaltsstoffe.

## Claims

1. Method for preparing a particulate free-flowing biomass comprising an oxidation-sensitive material of value, **characterized in that**, for preparing the particulate biomass, hydrophilic or hydrophobic silica is used in an amount such that a concentration of hydrophilic or hydrophobic silica in the particulate biomass of 0.1 to 8% by weight is established, wherein the product has a cell content of at least 60% by weight and wherein the cells of the biomass have a lipid content of at least 20% by weight, based on the cell dry matter.

2. Method according to Claim 1, **characterized in that** the hydrophilic silica has a specific surface area of 150 to 600 m²/g and a dioctyl adipate absorption value of 2 - 3.5 ml/g.

3. Method according to Claim 1, **characterized in that** the hydrophobic silica has a methanol wettability of at least 40%, preferably at least 45%, particularly preferably at least 50%.

4. Method according to any of the preceding claims, **characterized in that** the oxidation-sensitive material of value is an unsaturated fatty acid, preferably an omega-3 fatty acid or omega-6 fatty acid, particularly preferably DHA.

5. Method according to any of the preceding claims, **characterized in that** the hydrophilic or hydrophobic silica is used in an amount such that a concentration of silica in the particulate biomass of 0.2 to 6% by weight, preferably 0.5 to 2.5% by weight, is established.

6. Method according to any of the preceding claims, **characterized in that** the biomass comprises cells from the taxon Labyrinthulomycetes, in particular those from the family of Thraustochytriaceae, preferably those from the genera Thraustochytrium, Schizochytrium or Aurantiochytrium, particularly preferably those from the genus Aurantiochytrium.

7. Method according to any of the preceding claims, **characterized in that** the biomass, for the purpose of preparing the particulate composition, is used in the form of fermentation broth with a solids content of 10 to 50% by weight, preferably 20 to 40% by weight, and is converted to the particulate biomass by drying.

8. Method according to any of the preceding claims, **characterized in that** the biomass is dried by a thermal process, preferably by nozzle spray drying or spray granulation, and the silica is preferably added during the drying process.

9. Particulate free-flowing biomass comprising an oxidation-sensitive material of value, **characterized in that** it has a cell content of at least 60% by weight and contains hydrophilic or hydrophobic silica in an amount of 0.1 to 8% by weight, preferably 0.2 to 6% by weight, particularly preferably 0.5 to 2.5% by weight, wherein the cells of the biomass have a lipid content of at least 20% by weight, based on the cell dry matter, and wherein the biomass comprises cells from the taxon Labyrinthulomycetes.

10. Particulate biomass according to any of the preceding claims, **characterized in that** the hydrophilic silica has a specific surface area of 130 to 600 m2/g, preferably 160 to 550 m2/g, and a dioctyl adipate absorption value of 1.5 - 4.0 ml/g, preferably 2.0 to 3.2 ml/g.

11. Particulate biomass according to Claim 9, **characterized in that** the hydrophobic silica has a methanol wettability of at least 40%, preferably at least 45%, particularly preferably at least 50%.

12. Particulate biomass according to any of the preceding claims, **characterized in that** at least 80% by weight, particularly at least 90% by weight, preferably at least 95% by weight, of the particles have a particle size of 100 to 3500 micrometres, preferably 100 to 2500 micrometres and the particulate biomass is preferably free-flowing and low in dust.

13. Particulate biomass according to any of the preceding claims, **characterized in that** the biomass comprises cells from the family of Thraustochytriaceae, particularly preferably from the genera Thraustochytrium, Schizochytrium or Ulkenia.

14. Feedstuff or foodstuff containing a particulate biomass according to any of the preceding claims and also other feedstuff ingredients.

## Revendications

1. Procédé de fabrication d'une biomasse fluide particulaire, qui contient une matière de valeur sensible à l'oxydation, **caractérisé en ce que**, pour la fabrication de la biomasse particulaire, une silice hydrophile ou hydrophobe est utilisée en une quantité telle qu'une concentration en silice hydrophile ou hydrophobe de 0,1 à 8 % en poids s'ajuste dans la biomasse particulaire, le produit présentant une teneur en cellules d'au moins 60 % en poids et les cellules de la biomasse présentant une teneur en lipides d'au moins 20 % en poids, par rapport à la masse cellulaire sèche.

2. Procédé selon la revendication 1, **caractérisé en ce que** la silice hydrophile présente une surface spécifique de 150 à 600 m²/g et une valeur d'absorption de l'adipate de dioctyle de 2 à 3,5 ml/g.

3. Procédé selon la revendication 1, **caractérisé en ce que** la silice hydrophobe présente une aptitude au mouillage par le méthanol d'au moins 40 %, de préférence d'au moins 45 %, de manière particulièrement préférée d'au moins 50 %.

4. Procédé selon l'une quelconque des revendications précédentes, **caractérisé en ce que** la matière de valeur sensible à l'oxydation est un acide gras insaturé, de préférence un acide gras oméga 3 ou un acide gras oméga 6, de manière particulièrement préférée le DHA.

5. Procédé selon l'une quelconque des revendications précédentes, **caractérisé en ce que** la silice hydrophile ou hydrophobe est utilisée en une quantité telle qu'une concentration en silice de 0,2 à 6 % en poids, de préférence de 0,5 à 2,5 % en poids, s'ajuste dans la biomasse particulaire.

6. Procédé selon l'une quelconque des revendications précédentes, **caractérisé en ce que** la biomasse contient des cellules du taxon Labyrinthulomycetes, notamment de la famille des Thraustochytriaceae, de préférence des genres Thraustochytrium, Schizochytrium ou Aurantiochytrium, de manière particulièrement préférée du genre Aurantiochytrium.

7. Procédé selon l'une quelconque des revendications précédentes, **caractérisé en ce que** la biomasse est utilisée pour la fabrication de la composition particulaire sous la forme d'un bouillon de fermentation ayant une teneur en solides de 10 à 50 % en poids, de préférence de 20 à 40 % en poids, et transformée en la biomasse particulaire par séchage.

8. Procédé selon l'une quelconque des revendications précédentes, **caractérisé en ce que** le séchage de la biomasse a lieu par un procédé thermique, de préférence par séchage par pulvérisation en buse ou granulation par pulvérisation, et la silice est de préférence ajoutée pendant le procédé de séchage.

9. Biomasse fluide particulaire, qui contient une matière de valeur sensible à l'oxydation, **caractérisée en ce qu'**elle présente une teneur en cellules d'au moins 60 % en poids, et contient une silice hydrophile ou hydrophobe en une quantité de 0,1 à 8 % en poids, de préférence de 0,2 à 6 % en poids, de manière particulièrement préférée de 0,5 à 2,5 % en poids, les cellules de la biomasse présentant une teneur en lipides d'au moins 20 % en poids, par rapport à la masse cellulaire sèche, et la biomasse comprenant des cellules du taxon Labyrinthulomycetes.

10. Biomasse particulaire selon l'une quelconque des revendications précédentes, **caractérisée en ce que** la silice hydrophile présente une surface spécifique de 130 à 600 m²/g, de préférence de 160 à 550 m²/g, et une valeur d'absorption de l'adipate de dioctyle de 1,5 à 4,0 ml/g, de préférence de 2,0 à 3,2 ml/g.

11. Biomasse particulaire selon la revendication 9, **caractérisée en ce que** la silice hydrophobe présente une aptitude au mouillage par le méthanol d'au moins 40 %, de préférence d'au moins 45 %, de manière particulièrement préférée d'au moins 50 %.

12. Biomasse particulaire selon l'une quelconque des revendications précédentes, **caractérisée en ce qu'**au moins 80 % en poids, notamment au moins 90 % en poids, de préférence au moins 95 % en poids, des particules présentent une taille de particule de 100 à 3 500 micromètres, de préférence de 100 à 2 500 micromètres, et la biomasse particulaire est de préférence fluide et pauvre en poussière.

13. Biomasse particulaire selon l'une quelconque des revendications précédentes, **caractérisée en ce que** la biomasse contient des cellules de la famille des Thraustochytriaceae, de préférence des genres Thraustochytrium, Schizochytrium ou Ulkenia.

14. Fourrage ou aliment contenant une biomasse particulaire selon l'une quelconque des revendications précédentes, ainsi que d'autres constituants de fourrage.
